# EUROPEAN PATENT APPLICATION

(11) **EP 1 656 887 A1**
(43) Date of publication of application: **17.05.2006**
(21) Application number: 05018730.1
(22) Date of filing: 29.08.2005
(51) Int. Cl.: A61B 10/00

(54) **Container for suspension and filtration of stool**

(30) Priority: 15.11.2004 JP 2004330949
(71) Applicant: JAPAN as represented by PRESIDENT OF NATIONAL CANCER CENTER, Chuo-ku, Tokyo 104-0045 (JP); HITACHI, LTD., Chiyoda-ku Tokyo 100-8280 (JP)
(72) Inventor: Matsumura, Yasuhiro, Tokyo 152-0034 (JP); Matsushita, Hisayuki c/o National Cancer Center, Chiba 277-8577 (JP); Tsunoda, Hiroyuki c/o Hitachi, Ltd., 12th Floor, Chiyoda-ku, Tokyo 100-8220 (JP); Harada, Kunio c/o Hitachi, Ltd., 12th Floor, Chiyoda-ku, Tokyo 100-8220 (JP); Okano, Kazunori c/o Hitachi, Ltd., 12th Floor, Chiyoda-ku, Tokyo 100-8220 (JP); Nagai, Keiichi c/o Hitachi, Ltd., 12th Floor, Chiyoda-ku, Tokyo 100-8220 (JP)
(74) Representative: Strehl Schübel-Hopf & Partner

(57) **Abstract**

A container for the suspension and filtration of stool enables quick, simple, and safe collection of cancer cells separated in stool. The container comprises (a) a stool collection container 1, (c) a stool processing container main body 20, and (d) a pushing member 30. The stool collection container 1 comprises a syringe 2 capable of collecting 0.5 g or more of stool by being thrust into stool, a stool collecting opening, a handle 3 provided on the periphery of the syringe at the opposite end to the stool collecting opening, and a cap member 4 provided at the opposite end to the stool collecting opening. The stool processing container main body 20 comprises: a syringe storage portion 21 for storing the syringe; a suspension portion 22 connected to the syringe storage portion for suspending the stool; a filtrate receiving container 23 detachably connected to the suspension portion for receiving a filtrate of the stool that has been suspended and filtered; and a filter 26 provided at a connection portion between the suspension portion and the filtrate receiving container. The pushing member 30 is pressed to press or tear the cap member so as to move the stool collected in the syringe of the stool collection container into the suspension portion.

SEQUENCE LISTING

<110> HITACHI,LTD.

<120> CONTAINER FOR SUSPENSION AND FILTRATION OF STOOL

<130> H400576

<140>
<141>

<160> 2

<170> PatentIn Ver. 2.1

<210> 1
<211> 22
<212> DNA
<213> Artificial Sequence

<220>
<223> Description of Artificial Sequence:Primer

<400> 1
aaacacctca agttccaacc ac 22

<210> 2
<211> 23
<212> DNA

<213> Artificial Sequence

<220>
<223> Description of Artificial Sequence:Primer

<400> 2
ggtaattttg aagcagtgtg ggc                23

## Description

### CLAIM OF PRIORITY

The present application claims priority from Japanese application JP 2004-330949 filed on November 15, 2004, the content of which is hereby incorporated by reference into this application.

### BACKGROUND OF THE INVENTION

### Field of the Invention

The present invention relates to a stool collection kit for collecting stool used in colorectal cancer examinations, and to a stool processing kit for the suspension and filtration of collected stool.

### Background Art

Colorectal cancer is at the top of the list in terms of cancer mortality rates in Europe and the United States. In Japan, too, the number of colorectal cancer patients has been rapidly increasing in recent years. This is believed to be due to the shift in the dietary patterns of the Japanese towards more Westernized, meat-oriented dishes. About 60,000 Japanese are affected with colorectal cancer every year. This number represents the third largest number of deaths on an organ-to-organ basis, following stomach cancer and lung cancer, and the number is expected to rise in the future. Meanwhile, it is known that colorectal cancer is nearly 100% curable by surgery if detected in its early stages. It is for this reason that colorectal cancer is one of the items for early cancer screening, and a number of examination methods have been devised over the years.

Examples of examinations for the early detection of colorectal cancer include enema and endoscopic examination. In an enema examination, barium is injected into the large intestine and caused to attach to its mucosal surface so as to examine surface irregularities using an X-ray. In an endoscopic examination, the inside of the large intestine is directly examined using an endoscope. The endoscopic examination is highly sensitive and specific, particularly with respect to the detection of colorectal cancer. In addition, it is advantageous in that it is capable of removing early cancer or polyps in a precancerous stage.

However, these methods are costly and place a heavy burden on the patient. They are also associated with the possibility of complications. Further, the endoscopic examination requires a great deal of skill for operation, and facilities that can provide this type of examination are limited in number. Therefore, these methods are not suitable for colorectal cancer screening of members of the general public who have no symptoms.

In response, a fecal occult blood test, which is simple and not costly, is widely used as a primary screening method for colorectal cancer. In this test, the presence of hemoglobin in stool is detected to determine the presence or absence of bleeding in the intestines, so that the presence of colorectal cancer can be indirectly predicted.

Although the fecal occult blood test is a widely practiced method, some people voice doubts as to its efficacy. One reason is that the sensitivity of the test is low at approximately 25%, which means that the rate of overlooking colorectal cancer patients is high. In addition, its positive predictive value is low, and the percentage of actual colorectal cancer patients among the subjects that are determined to be positive in a fecal occult blood test is 10% or less. The test is thus fraught with many false positives, and there is a strong need to develop a more reliable and novel examination method.

As one such novel colorectal cancer examination method, a method utilizing cancer cells that have exfoliated into stool is gaining attention. As compared with the fecal occult blood test, whereby the bleeding in the intestines, which could be an indirect result of colorectal cancer, is examined, this method, which involves a direct examination concerning the presence of cancer cells, is expected to constitute a more reliable examination method.

In the method involving the utilization of separated cancer cells, two different strategies are conceivable. In one strategy, nucleic acid derived from cancer cells is directly extracted from the stool. In the other, cancer cells themselves are directly collected. The latter strategy of collecting cancer cells is advantageous in that cancer-cell derived products can be concentrated.

Examples of the method of collecting separated cancer cells in the large intestine include a method involving Percoll (Int.J.Cancer, Vol. 52, 347-350, 1992, Gastroenterology, Vol. 114, 1196-1205, 1998, and International Journal of Molecular Medicine, Vol. 13, 451-454, 2004), and a method whereby cancer cells are collected from the surface of frozen stool (WO97/09600, Clinical Cancer Research, Vol. 4, 337-342, 1998, The Lancet, Vol. 359, 1917-1919, 2002, and APMIS, Vol. 110, 239-246, 2002). A total stool processing system for the collection of cells from stool has also been devised by the present inventors (JP Patent Publication (Kokai) No. 2005-046065 A).

### SUMMARY OF THE INVENTION

In the existing methods for collecting colorectal cancer cells, stool is put in a bag together with a suspending solution so as to suspend the stool, and then the stool is crushed using a device called stomacher, which allows solid matter to be mildly crushed (WO97/09600 and JP Patent Publication (Kokai) No. 2005-046065 A). In JP Patent Publication (Kokai) No. 2005-046065 A, the stool suspension is further filtered with a funnel-type filter, and colorectal cancer cells are collected from the filtrate.

However, in these methods for collecting stool, the steps involving the stomacher or the filtering of the suspension involve open systems, and there are problems concerning odors in the surrounding areas and the scattering of the stool suspension. Further, because these methods involve many manually performed steps, they are not suitable for the processing of large volumes of specimens.

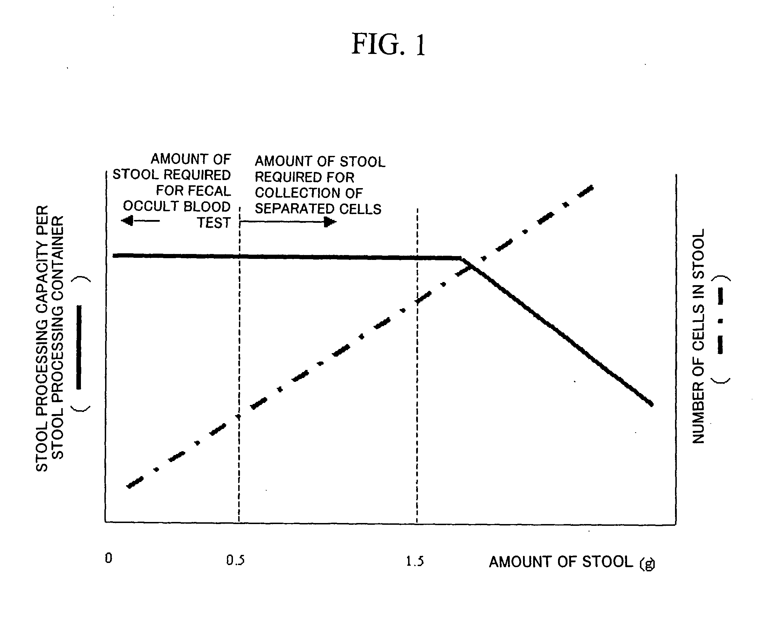

Furthermore, conventional stool-specimen collecting/suspending containers for the fecal occult blood test cannot handle the required volume of stool for cell recovery in the aforementioned method. Although the fecal occult blood test enables detection from a small amount of stool (0.1 g or less), at least 0.5 g of stool is required if cells are to be collected, as shown in Fig. 1.

It is therefore an object of the invention to provide a stool collection container and a stool processing apparatus for a quick, simple, and safe collection of exfoliated cancer cells in stool.

The invention achieves the aforementioned object through the use of a stool collection kit and a stool processing kit in which the process of collection, crushing, suspension, and filtration of stool can be performed in a streamlined manner.

Specifically, the stool collection kit of the invention comprises: (a) a stool collection container comprising a syringe, a handle, a coupling portion, and a cap member; and (b) a storage container comprising a storage cylinder having a coupling portion. The stool processing kit of the invention comprises: (a) a stool collection container comprising a syringe, a handle, a coupling portion, and a cap member; (c) a stool processing container main body comprising a syringe storage portion, a suspension portion, a filtrate receiving container, and a filter; and (d) a pushing member.

In the stool processing kit, the suspension portion, which is used for crushing and suspending stool, the filter, which is used for filtering the suspended stool, and the filtrate receiving container, which is used for recovering a filtrate through the filter, are integrally structured. Further, by attaching the stool collection container to the stool processing kit, the collected stool can be directly pushed into the stool processing kit main body.

The steps of crushing, suspension, and filtration are carried out simultaneously by shaking the stool processing kit. These steps can be automated by mounting the stool processing kit on a shaking device.

In accordance with the invention whereby the steps of collecting stool, crushing it, suspending it, and filtering it are carried out in an integral manner, the procedure for obtaining a filtrate of stool can be performed simply and in a short time. Further, because the suspension of stool is carried out in a closed system, the danger of sample scattering can be minimized, and the level of operator safety can be enhanced. In addition, a large volume of stool (0.5 g or more), which is necessary for the collection of cancer cells, can be processed. It is also possible to automate the aforementioned series of operations so as to process multiple specimens.

### BRIEF DESCRIPTION OF THE DRAWINGS

Fig. 1 shows a conceptual diagram of the relationship between the stool processing capacity of a stool processing container and the number of cells in the stool.
Fig. 2A and 2B show cross sections of a stool collection container according to the invention.
Fig. 3A to 3C show a procedure for suspension and filtration of stool using the stool collection container of the invention.
Fig. 4 shows a cross section of a stool processing container main body of the invention.
Fig. 5A to 5E show cross sections illustrating a procedure for processing stool using the stool collection container of the invention that is fitted in the stool processing container main body.
Fig. 6 schematically shows the process of magnetic separation, washing, and recovery of cancer cells from a stool filtrate.
Fig. 7A and 7B show cross sections of an improved stool collection container and stool collection container main body.

### DESCRIPTION OF THE PREFERRED EMBODIMENTS OF THE INVENTION

In the following, the concrete structures and methods of use of a stool collection kit and stool processing kit according to the invention will be described.

### (Stool collection)

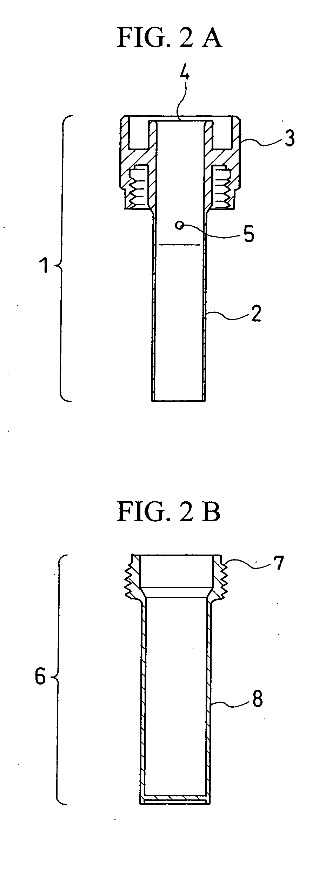

Fig. 2A and 2B show cross sections of a stool collection container portion and a storage container portion of a stool collection kit of the invention. A stool collection container 1 comprises a syringe 2 and a handle 3 with which the container can be held during use. The tip of the syringe 2, which is the part that is thrust into stool, has a hollow internal structure. The stool collection container 1 is sealed near the handle 3 of the syringe 2 with a rubber-like cap packing member 4 as a cap member of the container 1. The cap packing member 4 can be moved into the syringe 2 by pushing it from the outside. Alternatively, the cap member 4 may be substituted by a thin film that can be torn afterward. Such thin film can be affixed to the syringe 2 near the handle 3. Also near the handle 3 of the syringe 2, an air opening 5 is provided for facilitating the movement of stool when it is stored inside the syringe 2 during collection. The storage container 6 comprises a coupling portion 7 via which the container can be coupled with the stool collection container and a container cylinder 8 in which the stool collection container can be stored.

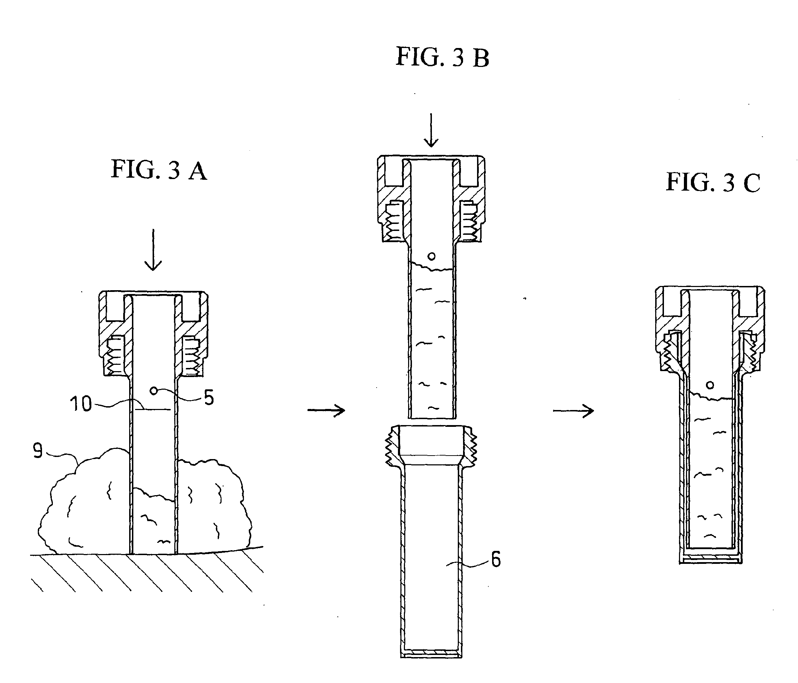

Fig. 3A shows how stool is collected. The tip of the syringe 2 of the stool collection container 1 is thrust into stool 9 so as to push the stool into the syringe 2. It is also possible to collect stool from a plurality of locations so as to collect a required amount of stool for cell examination, which is 0.5 g or more, by thrusting the collection container into the stool more than once. The air opening 5 functions to release the air inside the syringe as the stool enters it; the opening, however, is not absolutely required. The syringe is also provided with a level line 10 for allowing the amount of collected stool to be known. The level line 10 may be set as required depending on the amount to be collected.

Fig. 3B shows the stool collection container 1 with the collected stool in it being attached to the storage container 6. The storage container 6 is capable of storing the stool collection container 1 inside. The stool collection container 1 can be threaded into the storage container 6 such that the stool can be sealed in an airtight manner.

Fig. 3C shows the stool collection container 1 having been accommodated in the storage container 6, in which state the stool can be stored or transported for subsequent operations. For transportation, the stool collection kit of the invention can be put in a conventional envelope or the like for mail or courier service. It is also possible to use a box specifically designed for the stool collection container in which a refrigerating agent is disposed. Using such a dedicated box, which can be produced in conformity with the shape of the stool storage container, less space would be required for transporting a number of specimens.

### (Suspension and filtration of stool)

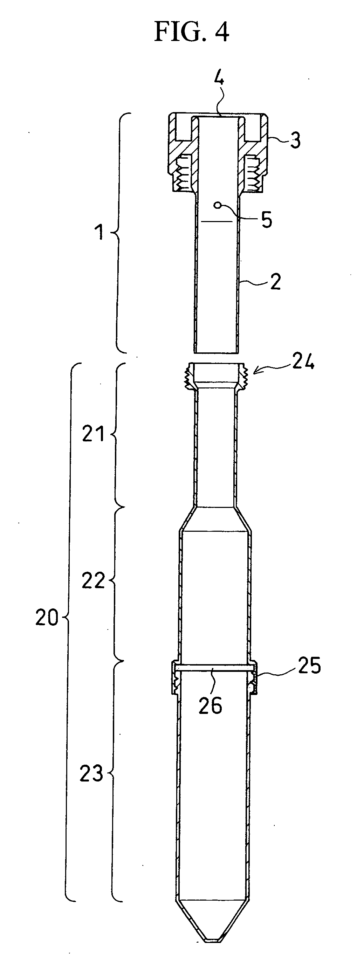

Fig. 4 shows a cross section of a main body 20 of the stool processing container. The main body 20 of the stool processing container comprises a syringe storage portion 21, a suspension portion 22, and a filtrate receiving container 23. At the top of the syringe storage portion 21, there is provided a coupling portion 24 for coupling with the stool collection container 1. The coupling portion 24 enables the stool collection container 1 to be coupled with the stool processing container main body 20 via a threaded connection. The container may be modified such that the stool alone can be dropped directly into the stool processing container main body 20 without coupling the stool collection container 1 with the main body 20. The syringe storage portion 21 is the part that accommodates the syringe 2 of the stool collection container 1. The suspension portion 22 is the central portion of the main body in which suspension of stool is carried out. The suspension portion 22 and the filtrate receiving container 23 are connected via a connection portion 25, which is a threaded portion comprising a portion in which a filter 26 is fitted and a coupling portion via which the filtrate receiving container 23 is connected. The filter 26 comprises a polyamide meshed filter with a diameter of approximately 30 mm for filtering the suspension. The filter 26 has a ring structure with a rubber periphery and is fitted into the connection portion 25 of the suspension portion 22. The material of the filter is not limited to the above-mentioned material and may be any other material as long as it satisfies the required filter conditions and operations. The diameter of the filter is preferably from about 500 µm to about 1000 µm; however, it may vary from about 100 µm to about 2000 µm depending on various conditions such as the amount of stool or the kinds of substances mixed with the stool. The filtrate receiving container 23 is threaded into the connection portion 25 for connection. As the filtrate receiving container 23, a 50 ml centrifugal tube referred to as "a conical tube" commercially available from Falcon Corporation, or a newly prepared tube may be used.

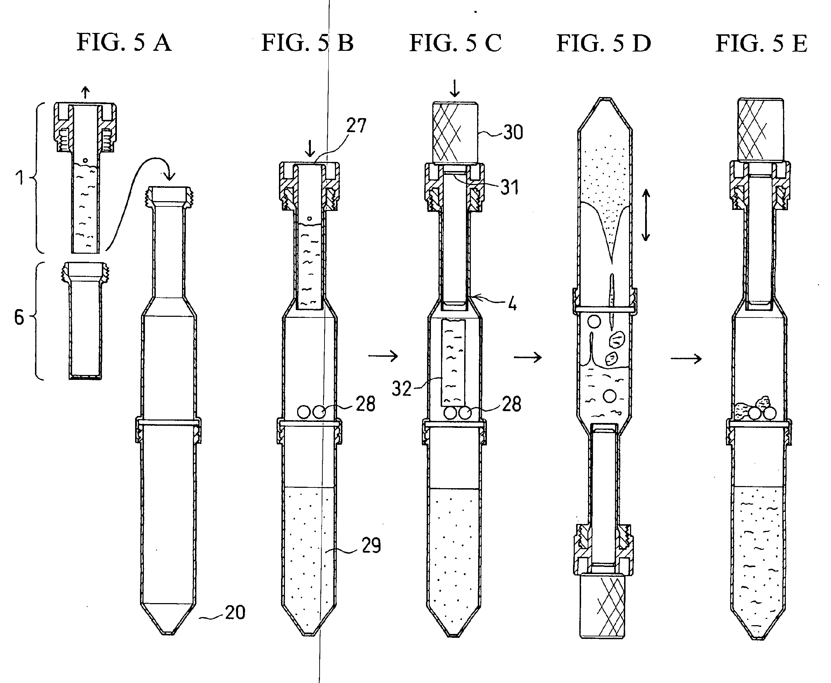

Fig. 5A shows the stool collection container 1 with the collected stool in it being taken out of the storage container 6.

Fig. 5B shows cross sections illustrating how the stool collection container 1 is fitted in the stool processing container main body 20 shown in Fig. 4. A rubber packing member 27 functions as the cap of the stool collection container 1. Inside the suspension portion 22, there are stool crushing balls 28 that may be made of ceramics, such as zirconia, or a metal material such as stainless steel. The crushing balls 28 preferably have a diameter of approximately 10 mm; however, it may range from about 5 mm to about 25 mm. The number of the crushing balls is normally 3, but may range from one to a dozen or so. In the filtrate receiving container 23, a suspension solution 29 is disposed in advance, the amount of which is preferably from about 20 ml to about 40 ml. The type of solution is not limited and any solution used for the washing or culturing of cells can be used, such as Hank's solution or DMEM culture solution. A substance that stabilizes the cells, such as blood serum or BSA, may be added to the solution.

Fig. 5C shows a cross section illustrating how the collected stool is pushed by a pushing bar 30 into the suspension portion 22. By pushing the pushing bar 30 into the syringe 2, the stool inside the stool collection container 1 can be pushed out into the suspension portion 22 of the stool processing container 20. A packing member 31 is fitted on the periphery of the axle of the pushing bar 30, whereby the pushing bar 30 and the syringe 2 can be hermetically sealed, the pushing bar 30 can be stabilized, and the leakage of the suspension can be prevented. By increasing the number of the packing members 31, a more hermetically sealed structure can be obtained. Alternatively, the pushing bar 30 may be fitted into the syringe storage portion 21 and fixed therein. The packing member 4 functioning as the cap of the stool collection container 1 moves towards the tip of the syringe 2 as the pushing bar 30 is inserted, thereby pushing out the stool. The stool 32 that has been pushed out into the suspension portion 22 is then crushed, suspended, and filtered.

Fig. 5D shows how the stool is suspended by holding the stool processing container 20 upside down and shaking it. At the center of the suspension portion 22, the stool and the crushing balls 33 are mixed with the suspension solution 29, such that the stool can be crushed. By repeating the shaking operations, the stool can be better suspended. Although in the drawing the stool processing container 20 is shaken while upside-down with the filtrate receiving container 23 facing upward, this is only an example and the direction of the stool processing container 20 may be changed as required depending on the state of the stool suspension. The stool processing container 20 is shaken by performing about 100 back-and-forth movements by hand or with a shaker so as to suspend the stool. The number of repetitions of such shaking movements may be varied from 50 to 1000 depending on the state of the stool. For the shaking operation, a commercially available shaker or a shaking device with an arm, for example, may be used. A meshed structure may be placed at the center of the stool processing container 20. In this way, the suspension of the stool can be promoted by friction, and further, some of solid crushed material in the solution can be captured by the mesh so that clogging of the filer can be reduced.

Fig. 5E shows how the suspension is filtered and moved into the filtrate receiving container 23. After the shaking operation, the stool processing container 20 is positioned with the filtrate receiving container 23 placed at the bottom, so as to collect the filtrate that has passed through the filter 26. In this state, most of the filtrate does not move downward and instead remain inside the suspension portion 22. Therefore, the suspension portion 22 is held with one hand, and the filtrate receiving container 23 is lightly struck with the other hand in a rhythmical motion, thereby causing the suspension to be collected in the filtrate receiving container 23. Alternatively, the main body 20 of the stool processing container may be held with one hand such that the filtrate receiving container 23 is located thereabove, and the main body 20 may be shaken in a top-to-bottom motion similar to the manner in which one would shake a thermometer, thereby causing the suspension to be collected in the filtrate receiving container 23.

The filtrate receiving container 23 is then removed from the main body 20 of the stool processing container 20, and the filtrate inside is transferred to another container. Cells are then collected from the filtrate and used for screening for colorectal cancer.

### (Cell collection using magnetic beads)

Cancer cells contained in the filtrate are collected using a carrier with an affinity to cancer cells. For the carrier, a magnetic bead with an antibody having an affinity to cancer cells bound to the surface thereof is used. Specifically, Ber-EP4 antibody-binding magnetic beads (Dynabeads Epithelial Enrich, Dynal) commercially available from Dynal are used. Other than Ber-EP4, antibodies that have an affinity to colorectal cancer cells can be used. Other than antibodies, aptamers or ligands with an affinity to colorectal cancer cells may be used.

40 µl of magnetic beads is added per tube containing about 20 to 40 ml of dispensed filtrate. The amount of the magnetic beads may be varied from about 20 to 400 µl.

The filtrate to which the magnetic beads have been added is then mixed using a mixing rotor so as to cause the cells in the filtrate to be bound to the magnetic beads. The mixing is preferably performed at room temperature or in a cold room at a temperature of 4°C, preferably for a period of 30 minutes or longer. This magnetic bead reacting step was performed at room temperature.

The tube with the thus mixed filtrate inside is then installed on a magnetic stand and shaken for 15 minutes, thereby collecting the magnetic beads on the side of the tube. The shaking is preferably performed for at least 10 minutes with a see-saw motion, rotation, or spinning, or under any other condition in which the filtrate can be gradually mixed.

After the magnetic beads have become attached to the wall surfaces, the filtrate is removed. After the filtrate has been removed, the tube is removed from the magnetic stand, washed with the aforementioned buffer solution, and the bead-washing solution is then collected. The amount of the buffer solution is 500 µl per tube; the amount, however, may be varied as required in view of the subsequent experiments. This step of magnetic separation was carried out at room temperature.

The washing solution is collected in an Eppendorf tube or the like that is smaller than the previously used tube. The tube with the washing solution therein is immediately installed on a dedicated magnetic stand. After the magnetic beads are collected on the side walls of the Eppendorf tube, the supernatant is removed, thereby obtaining pellets of cell-bead complex. The magnetic separation step and the step involving the Eppendorf tube were conducted at room temperature.

### (Colorectal cancer diagnosis)

The pellets collected in the above-described standardized protocol are used as a specimen for the determination of colorectal cancer. For the determination of cancer, cells themselves may be utilized, or a substance extracted from the cells may be utilized. When cells themselves are utilized, the pellets can be used immediately after collection. They may also be preserved using a cell fixation solution. When an extracted substance is utilized, the pellets can be frozen and preserved at -80°C.

When cells themselves are utilized, the cells are stained by Papanicolaou stain and then observed via a microscope. If the ratio of nucleus to cytoplasm (N/C ratio) is high and atypical cells with an aggregation of chromatin are observed, the cells are determined to be cancerous. Any other staining method may be used as long as it is capable of identifying cancer cells. Other than general staining methods, immunostaining procedures utilizing cancer cell-specific antibodies may be applied.

DNA or RNA may be extracted from the cells and used for cancer determination. For the extraction of DNA or RNA, various nucleic acid extraction kits commercially available form a number of companies may be employed. Examples include Dynabeads DNA DIREIC Universal from Dynal, QIAampDNA Mini Kit from QUIAGEN, and SepaGene from Sanko Junyaku Co., Ltd. For the extraction of RNA, ISOGEN from Nippon Gene Co., Ltd., and TRIzol Reagent from Invitrogen Corporation may be used.

In contrast to the colorectal cancer cell collection protocol employing the stool collection kit and the stool processing kit according to the invention, the conventional stomacher method for the collection of colorectal cancer cells differs in terms of the procedures for the collection of a specimen and filtration. Namely, in accordance with the invention, collection of stool is carried out using a specific stool collection container, and the suspension and filtration are performed without the use of a stomacher but with a stool processing container that can be coupled with the stool collection container. Because suspension and filtration can be performed in the same step using coupled containers, the burden of having to deal with stool can be eliminated, the operation time can be reduced, and the volume of expendable articles can be reduced. Further, due to the use of stainless-steel or ceramic balls for crushing stool, the efficiency of operation can be improved.

### Examples

Hereafter the invention will be described in greater detail with reference to examples. It is to be noted, however, that the invention is not limited to any of these examples.

### (Example 1: Preparation of the stool collection container)

Individual resin components of the stool collection kit and the stool processing kit of the invention were prepared by injection molding. The components include a stool collection container (made of polyethylene), a storage container (made of polyethylene), a stool processing kit main body (made of polyethylene), a filter (with the peripheral portion made of elastomer, and the filter portion made of nylon), a cap packing member (made of elastomer), and a pushing bar (made of polyethylene). The foregoing descriptions should be referred to for the descriptions of each of these components.

### (Example 2: Evaluation based on an imitation stool)

Using imitation stool, methods of use of the stool collection container and the stool processing container were analyzed. The imitation stool was prepared by mixing 2.5 g of fishing bait ("Ukigoi" from Marukyu K.K.) as a base material and 1 g of flour ("Camellia" strong flour from Nisshin Flour Milling Co., Ltd.) as a thickener. 2.5 ml of water was then added to the mixture and kneaded.

Using the thus prepared imitation stool, an appropriate amount of stool for a single stool collection container was determined. Initially, three different amounts were collected from the imitation stool using the stool collection containers, namely a half-thrust portion (0.82 g), a one-thrust portion (1.36 g), and a two-thrust portion (2.87 g). Each stool collection container was then set on the stool processing container main body, and the imitation stool was pushed into the suspension portion using the pushing bar. Then, while the pushing bar was held by the fingers, the stool processing kit was moved up and down in a 100 reciprocating movements so as to crush and suspend the imitation stool. Thereafter, the stool processing kit was allowed to stand while the movement of the suspension into the filtrate receiving container was examined.

The results showed that the suspension passed through the filter without difficulty in the cases of the imitation stool of the amounts corresponding to the half-thrust and the one thrust of the stool collection container. However, the filter became clogged in the case of the two-thrust amount of the imitation stool, resulting in a decrease in the filtrate amount. Further, in the case of the two-thrust amount, there was a great deal of sediment mixed in the filtrate, indicating that this amount was excessive for the container used. Therefore, it was thought that a preferable amount of stool to be collected using the stool collection container was not more than the amount corresponding to a single thrust (approximately 1.5 g) of the stool collection container.

### (Example 3: Collection of cells from a colorectal cancer patient)

Three stool specimens (A, B, and C) were obtained from three colorectal cancer patients prior to surgery. Regarding the use of the stool specimens, each patient was informed in advance about the nature of the experiment and their prior consent was obtained.

Using the stool collection containers, a one-thrust portion of stool was obtained from each of the patients. Each container in which the stool was collected was coupled with the stool processing container main body. 30 ml of Hanks' solution + 10 % FBS was disposed in the filtrate-receiving portion of each container in advance.

Then, using the pushing bar, the stool was pushed out into the stool processing container main body. The container was then shaken in 100 reciprocating movements while pressing the pushing bar so as to prevent it from becoming detached. As a result, the stool was crushed and turned into a suspension. Some of the suspension was filtered through the filter positioned at the boundary between the main body and the filtrate receiving container and moved to the filtrate receiving container below. After the shaking operation, the stool suspension remaining in the suspension portion was moved to the filtrate receiving container by vibrating the container by lightly striking it with fingers.

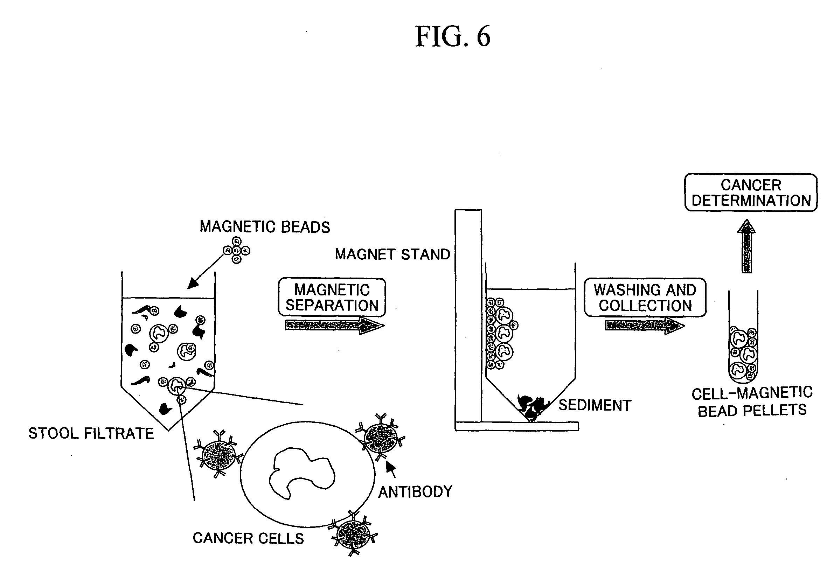

The filtrate receiving container was then removed from the stool processing container main body, and the filtrate was transferred into a new 50 ml centrifugal tube (IWAKI). To the filtrate was added 40 µl of Ber-EP4 antibody-binding magnetic beads (Dynabeads Epithelial Enrich available from Dynal), and the mixture was stirred using a mixing rotor (VMR-5 available from AS ONE) for 30 minutes. After stirring, the centrifugal tube was set on a magnet stand (Dynal MPC-1 available from Dynal) and shaken for 15 minutes using a Mild Mixer (SI-36 available from TAITEC), thereby collecting the magnetic beads on the wall surfaces of the magnet stand. After the filtrate was removed, the centrifugal tube was removed from the stand, 500 µl of PBS solution was added to each centrifugal tube as a washing solution, and the beads collected on the wall surfaces were washed therewith. The washing solution containing the beads was collected in an Eppendorf tube (1.5 ml), which was then set on the magnet stand (Dynal MPC-S available from Dynal), and the magnetic beads were collected on the side walls of the Eppendorf tube. Finally, the washing solution was removed, thereby obtaining pellets of a cell-bead complex. Fig. 6 schematically shows the procedure from the filtering of stool to the washing and collection.

Cell staining was performed using some of the cell-bead complex pellets from patient A. After 100 µl of YM fixing solution was added to the pellets and the pellets were suspended, the suspension was transferred to a 50-ml centrifugal tube, where YM fixing solution was further added until a total amount of 25 ml was obtained, thereby preparing a cell-containing fixing solution. The cell-containing fixing solution was then dispensed into an automatic smearing apparatus for 8 slide glasses. After YM fixing solution was further added and the apparatus was filled with the solution, the solution was centrifuged at 2000 rpm for 10 minutes, thereby smearing the slide glasses with the cells. The slides were then dried via cold blasts of air, and the cells were fixed with 95 % ethanol. The cells were then dyed by Papanicolaou stain, which is a typical staining method for cell morphology observation purposes, and then microscopically observed.

As a result, it was observed that the pellets contained cells that could be judged to be of the epithelial origin, thus indicating that cells can be recovered from stool in accordance with the method of the invention.

### (Comparative Example: Collection of cells from stool by a conventional method)

Using the same stool samples obtained from the aforementioned three colorectal cancer patients, cell-magnetic bead pellets were collected by a conventional method using a stomacher (JP Patent Publication (Kokai) No. 2005-046065 A). Some of the sample from patient A was stained in the same manner as in Example 3, and the cells were observed. As a result, it was confirmed that cells of epithelial origin were present. When the cells were observed in greater detail morphologically, nuclei of various sizes, concentration of chromatin, and the thickening of nuclear membrane were observed, which were indicative of the fact that the cells were Class V colorectal cancer cells. Thus, it was clearly shown that cancer cells were present in the stool from patient A.

However, as compared with the case of cells collected by the method of the invention, the cells were observed to include many lymphoid cells.

### (Example 4: Sequence analysis of DNA derived from the collected cells)

DNA was extracted from the collected magnetic bead pellets using a SepaGene Kit (available from Sanko Junyaku Co., Ltd.). Using the obtained DNA as a template, a region included in exon 15 of the APC (adenomatous polyposis coli), which is a cancer suppressor gene, was amplified by PCR. The sequences of the primer used were F: 5 '-AAACACCTCAAGTTCCAACCAC-3' and R: 5'-GGTAATTTTGAAGCAGTCTGGGC-3', and the number of bases in the amplified region was 1553 bp. A region of the thus amplified segment called MCR (mutation cluster region, codon 1250-1550), which is a hot spot for APC gene mutation, was sequenced. For the sequence analysis, a Big Dye Terminator v3.1/1.1 cycle kit (ABI) was used in accordance with the attached protocol.

As a result, a nonsense mutation (Lys → Stop/cdon1308) was found in sample A in which the 3940^{th} A in the APC gene (M74088) had changed into T. In sample B, two kinds of nonsense mutations were found; namely, one (Glu → Stop/cdon1322) in which the 3982th G had changed into T, and the other (Cys → Stop) in which the 4179^{th} T had changed into A. In sample C, a nonsense mutation (Gln → Stop/cdon1406) was found in which the 4234^{th} C had changed into T.

On the other hand, DNA was similarly extracted from the cells collected by the conventional method, and a sequence analysis was performed. As a result, in sample A, a nonsense mutation (Lys → Stop/cdon1308) was found in which the 3940^{th} A of an APC gene (M74088) had changed into T. In sample B, a nonsense mutation (Glu → Stop/cdon1322) was found in which the 3982th G had changed into T. In sample C, no mutation was determined.

Thus, it was shown that the cells collected in accordance with the invention have less contamination of lymphoid cells, and, possibly, the cells are cancer-cell rich, which are useful in determining cancer cells by sequence analysis.

### (Example 5: Preparation of an improved container)

The stool processing kit was improved to provide better operability.

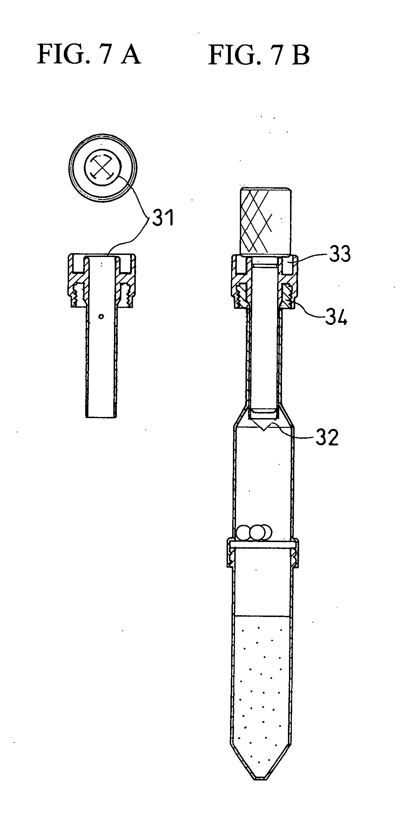

Fig. 7A and 7B show cross sections of an improved stool collection container and stool processing container main body. Fig. 7A shows the stool collection container, which has a thin film 31 attached instead of the cap packing member. Fig. 7B shows a cross section of the stool processing container main body to which the stool collection container and a pushing bar were attached. A tip 32 of the pushing bar was sharply formed so that it could penetrate the thin film 31. After tearing the thin film 31, the pushing bar could be pressed so as to push the stool inside the syringe directly into the stool processing container main body.

A coupling portion 33 was provided so that the pushing bar and the main body could be fixed to each other. As a result, it was possible to prevent the pushing bar from being displaced during the shaking of the stool processing container main body.

An inlet portion 34 of the stool processing container main body was modified to result in a wider structure than those of conventional containers. As a result, the mounting of the collecting container on the main body was facilitated, and the attachment of stool to the inlet portion of the main-body container during the mounting of the collecting container due to jiggling of the hand was prevented.

In accordance with the stool collection kit and the stool processing kit of the invention, the collection of cancer cells exfoliated inside the large intestine is facilitated, thereby contributing to the early detection of colorectal cancer.

## Claims

1. A stool collection kit comprising a stool collection container (1) and a storage container (6) for storing said stool collection container, wherein:
(a) said stool collection container (1) comprises a syringe (2) capable of collecting 0.5 g or more of stool by being thrust into stool; a stool collecting opening; a handle (3) provided on the periphery of said syringe (2) at the end opposite from said stool collecting opening; one coupling portion that can be coupled with said storage container (6); and a cap member (4) provided at the other end of said stool collecting opening; and
(b) said storage container (6) comprises the other coupling portion (7) that can be coupled with said stool collection container (1), said storage container comprising a storage cylinder (8) in which said stool collection container can be stored.

2. The kit of claim 1, wherein said syringe (2) comprises an air opening (5) via which air inside said syringe can be released during the collection of stool.

3. The kit of claim 1, wherein said syringe (2) is made of a transparent or semitransparent material and comprises a level line (10) indicating the amount of stool collected.

4. The kit of claim 1, wherein said cap member (4) comprises
a cap packing member that can be moved inside said syringe (2) as it is pushed by a pushing member (30), or
a thin film (31) for capping purposes that can be torn by a pushing member (30) with a pointed tip (32).

5. The kit of claim 1, further comprising a cooling material or a cooling unit for storage or transportation purposes.

6. A stool processing kit comprising a stool collection container (1), a stool processing container main body (20) that can be coupled with said stool collection container, and a pushing bar (30) for moving collected stool from said stool collection container into said stool processing container main body, wherein:
(a) said stool processing container comprises a syringe (2) capable of collecting 0.5 g or more of stool by being thrust into stool; a stool collecting opening; a handle (3) provided on the periphery of said syringe (2) at the end opposite from said stool collecting opening; and a cap member (4) provided on the end opposite from said stool collecting opening;
(c) said stool processing container main body (20) comprises: a syringe storage portion (21) for storing said syringe (2); a suspension portion (22) connected to said syringe storage portion for suspending stool; a filtrate receiving container (23) that is detachably connected to said suspension portion, said filtrate receiving container receiving a filtrate of the suspended and filtered stool; and a filter (26) provided at a connection portion (25) between said suspension portion (22) and said filtrate receiving container (23); and
(d) said pushing member (30) is adapted to move the stool collected in said syringe (2) of said stool collection container (1) into said suspension portion (22) by pressing or tearing said cap member (4).

7. The kit of claim 6, wherein a crushing means (28) for crushing the stool is provided inside said suspension portion (22), said crushing means preferably comprising one or more balls of metal, ceramic, glass, or plastic material.

8. The kit of claim 6, wherein said filter (26) comprises a polyamide mesh.

9. The kit of claim 6, wherein a stool-suspending solution (29) is contained in said filtrate receiving container (23), said stool suspending solution preferably comprising a cell washing solution and/or a cell culture solution.

10. The kit of claim 6, wherein said stool collection container (1) comprises one coupling portion that can be coupled with said syringe storage portion (21) of said stool processing container, wherein said syringe storage portion of said stool processing container comprises the other coupling portion (24) that can be coupled with said stool collection container.

11. The kit of claim 6, wherein said suspension portion (22) of said stool processing container main body (20) comprises one coupling portion that can be coupled with said filtrate receiving container (23), and wherein said filtrate receiving container comprises the other coupling portion that can be coupled with said suspension portion.

12. The kit of any of claims 1, 6 and 11, wherein said coupling portions comprise threaded portions or snap-fit portions.

13. The kit of claim 6, wherein said syringe storage portion (21), said suspension portion (22), and said filtrate receiving container connection portion (25) of said stool processing container main body (20) are made of a transparent or semi-transparent material.
